# EUROPEAN PATENT APPLICATION

(11) **EP 2 335 562 A1**
(43) Date of publication of application: **22.06.2011**
(21) Application number: 09179346.3
(22) Date of filing: 15.12.2009
(51) Int. Cl.: A61B 5/00, A63B 71/08, A61B 1/24

(54) **Intra-oral container for accommodating an electronic device**

(71) Applicant: Concept/One B.V., 1272 PM Huizen (NL)
(72) Inventor: Messie, Frank, 1272 PM Huizen (NL)
(74) Representative: Hatzmann, Martin

(57) **Abstract**

The invention relates to an intra-oral container comprising at least one region for accommodating an electronic device, wherein the container is curved and is shaped to be fitted in at least a portion of a space formed by a lower jaw of a recipient, wherein at least a portion of the container is displaceable for enabling compression of the container during insertion thereof in said space.

## Description

### FIELD OF THE INVENTION

The invention relates to an intra-oral container for accommodating an electronic device.

### BACKGROUND OF THE INVENTION

In a clinical set-up, for example during an intensive care of a patient, there is a need for non-invasive measurements and monitoring of physiological parameters of the patient, which provides necessary insights in a medical status of the patient.

An embodiment of the intra-oral container as is set forth in the opening paragraph is known from US2009210032. The known device is arranged to accommodate a suitable electronic device, which can be used, for example, for monitoring, diagnosing or treating local and/or systemic diseases and conditions of humans or animals.

The known container is arranged to be positioned inside the mouth of the recipient externally to the teeth. In order to enable a positional stability of the known container it comprises a projection conceived to engage with a mole of the recipient.

It is a disadvantage of the known container that it is not convenient for use for purposes of prolonged monitoring, for example in an intensive care department. It is a further disadvantage of the known container that it has to be fixed to a molar of the recipient thereby possibly inducing tissue damage to a gum and/or to enamel.

A further embodiment of the container as is set forth in the opening paragraph is known from JP7246191. The known container is arranged to engage with the upper teeth of the recipient at a frontal region and at two lateral regions thereof for enabling positional consistency of the container and an electronic device associated with it.

It is a disadvantage of the known container that it may be not convenient for durable measurements as a substantially massive container has to be positioned adjacent to the palate, which may obstruct an air path during inhalation and exhalation. In addition, due to the fact that the known container is conceived to engage the teeth along a substantial surface area hygienic problems may occur due to obstruction of these teeth for cleaning.

A still further embodiment of the container as is set forth in the opening paragraph is known from US 20031544990. The known container is arranged as a bite, like one used by sportsmen, wherein both the upper teeth and the lower teeth have to engage the container and to substantially constantly exhibit a biting force on it.

It is a disadvantage of the known container that it is not suitable for being used for prolonged monitoring, especially during intensive care as the teeth have to be constantly engaged with the bite causing the mouth to be kept shut. Clearly, such posture may not be kept for several hours without interruption.

### SUMMARY OF THE INVENTION

It is an object of the invention to provide a container for accommodating an electronic device for enabling intra-oral monitoring or therapy, wherein such container is user friendly, easy to handle and is suitable to be used during prolonged monitoring, especially under the intensive care conditions.

To this end the intra-oral container according to an aspect of the invention comprises at least one region for accommodating an electronic device, wherein the container is curved and is shaped to be fitted in at least a portion of a space formed by a lower jaw of a recipient, wherein a portion of the container is displaceable for enabling compression of the container during insertion thereof in said space.

The insight of the invention is based on the fact that during clinical studies it has occurred that the lower portion of the oral cavity, i.e. an intro-oral space inside the lower jaw is particularly suitable for allowing a durable dwelling of a monitoring device. This space has an advantage that it is easily accessible and that a device positioned in this cavity does not interfere with a physiologic activity of a recipient, it does not obstruct breathing, it can be left in the oral cavity during eating and it does not hamper mouth hygiene.

When such container is used for enabling durable monitoring critical processes which may take place in the recipient, for example, in a patient being under intensive care, the patient may be readily monitored improving quality of the medical care.

Preferably, the region arranged for accommodating an electronic device comprises a cavity wherein a suitable device may be housed. The device may be positioned inside this cavity before use and may be properly sealed. Alternatively, the region may be implemented as a suitable compartment for accommodating an electronic device. Preferred embodiments of the electronic device relate to a monitoring device, such as a temperature or a CO2 sensor, a stimulating device comprising electrodes or a micro-pump adapted to collect a suitable volume of saliva, for example 0.1 ml, for further analysis. The electronic device may also relate to a microscopic camera. The electronic device may be suitably sealed for protecting its integrity while dwelling in the space of a lower jaw of the recipient.

It is possible that both the container and the electronic device are reusable or disposable. It is also possible that the container is disposable and that the electronic device is reusable.

Preferably, the container is U-shaped, arc-shaped or O-shaped.

It will be appreciated that for different purposes differently shaped containers may be required. For example, for an application wherein one or two devices are to be accommodated by the container an arc-shaped container may suffice, wherein for an application wherein a greater plurality of devices may be required a U-shaped or an O-shaped container may be required.

It will be appreciated that it is either possible to manufacture a set of suitable shapes, wherein a smaller one may correspond to an infant, a medium to an average value for females and a large corresponding to an average value for males. It is also possible to provide a differently arranged set of shapes, like a kit comprising an arc, a U-shape and an O-shape, which may be selectable on demand.

It will be further appreciated that according to the invention the container is arranged for allowing at least a partial compression thereof for inserting into the cavity formed by the lower jaw. Accordingly, an arc-shaped container may be formed such that a radius of the arc is reduced during insertion. The O-shaped container may be adapted to allow compression of the O-shape in one or more directions during insertion. It will be further appreciated that the O-shape may relate to a circular or an oval container. The U-shaped container is preferably adapted to allow compression of the legs of the U-shape with respect to the base of the U-shape, preferably towards a symmetry direction of the U-shape. Accordingly, by providing for the compression of the container handling thereof during insertion into the oral cavity is substantially improved. Expansion of the container against the inner side of the lower jaw enables positioning and fixation of said container.

For example, a U-shape may be provided with one or two displaceable legs, in the latter case handling of the U-shape is substantially improved. Preferably, the leg of the U-shape is displaced towards a symmetry axis of the U-shape resulting in the U-shape compressed as a whole which simplifies its insertion into the mouth of a recipient and enables a suitable fixed positioning inside the lower jaw.

It will be appreciated that the term 'lower jaw' relates to both the bony structures and the soft tissue as usually present in a three-dimensional space under the tongue. It will be further appreciated that it is not necessary that all teeth are present in the jaw for practicing the invention.

In a still further embodiment of the container according to the invention, the container, is adapted to exhibit a lateral force on the lower jaw when positioned in said space. For example, the legs of the U-shape may be dimensioned to allow the U-shape to firmly dwell in the lower jaw.

It is found that it is advantageous to shape and to dimension the container so that it, after being placed in the space inside the lower jaw, substantially firmly abuts against the lower jaw. For example, it may be advantageous to provide the U-shape with slightly diverging legs opting that at least the distal portion of the leg exhibits a lateral force against the lower jaw.

In an embodiment of the container according to the invention both legs of the U-shape are displaceable. It is found that when both legs are displaceable a better compression of the U-shape may be achieved leading to a further improvement of the handling properties of the container according to the invention.

It is found to be particularly advantageous to provide the U-shape with a length between 25 — 30 mm (i.e. the distance between the base of the U-shape and the distal portions of the legs). A width of the U-shape may be set to a value in the range of 32 - 36 mm, when at rest. Preferably, a width of the compressed U-shape is 20 - 25 mm. It will be appreciated that the dimensions of the O-shaped or an arc-shaped containers are similar.

In a still further embodiment there may be a section of material between the outer parts of the container, comprising a thin, flat, flexible material with a closed top layer and a perforated bottom layer enwrapping a space which, when vacuum is applied to it, will suck against the bottom of the oral cavity, preventing the container to move.

In a still further embodiment of the container according to the invention, the container is substantially three-dimensional, a curvature of an outer surface of the container substantially matching a curvature of the space formed by the lower jaw.

It is found to be particularly advantageous to provide the container with a pre-determined outer slope which substantially matches the slope of the lower jaw. The combination of said slope with the capability to expand the container against the inner lower jaw, will substantially improve the firm positioning of the container.

In a still further embodiment of the container according the invention at least the outer surface of the container conceived to come into contact with the lower jaw comprises a soft material.

It is found that in order to prevent soft tissue damage and to improve positioning of the container inside the lower jaw, at least a surface conceived to come into contact with a tissue of the lower jaw (soft tissue or bony tissue) may be made soft. Preferably, the soft material is thermoplastic. It is found to be advantageous to provide at least a portion of the container conceived to contact with the lower jaw of the recipient with the soft material, which may be thermosettable when heated to about 36 degrees Celsius. It will be appreciated that a person skilled in the art will know which class of materials may meet these criteria. It will be further appreciated that it is not necessary that the thermosetting material hardens completely, as this may introduce some discomfort to the soft tissues of the oral cavity. It will be understood, that the container may be manufactured entirely from a substantially soft and, preferably, flexible material. Alternatively, only the surface conceived to abut the lower jaw may be manufactured form a soft and, preferably, flexible material. Suitable soft materials comprise but are not limited to biocompatible resins, foams, gels or the like.

In a still further embodiment of the container according to the invention the soft material is shapeable.

It is found to be particularly advantageous to provide the surface of the container, for example, the U-shape conceived to come into contact with the lower jaw from a soft, shapeable material. This has an advantage that the container may three-dimensionally fit into the lower jaw substantially without areas which are over-pressured and areas which have no contact with the jaw. Preferably, the soft material comprises a bag provided with a filling, a shape of the bag being vacuum-shapeable. Those skilled in the art readily appreciate that a suitable bag, for example, filled with foam particles may be readily shaped in three dimensions using vacuum.

In a still further embodiment of the container according to the invention, the container is at least partially implemented from a resilient material. For example, referring to the U-shape, at least one leg or the area between the legs may be resilient.

It is found that the container may be advantageously manufactured from a resilient material, such as a biocompatible rubber or the like. Preferably, the container is molded, which enables its simple production.

In a still further embodiment of the container according to the invention the at least one region for accommodating an electronic device comprises a cavity for receiving said device.

In a still further embodiment of the container according to the invention, the container is provided with a projection, cavity or a rim for enabling handling of the container.

It is found to be particularly advantageous to provide the container with an element which can be engaged by a medical specialist while inserting it in the lower jaw. Due to the fact that the container is usually handled using tweezers, engagement with such element may reduce a tissue damage hazard compared to a situation when the container is to be handled by engaging its outer surface.

In a still further embodiment of the container according to the invention it further comprises at least one cavity for enabling positioning of the container using vacuum suction.

It is found that positioning of the container in the lower jaw may be improved when vacuum suction is used. For this purpose the container may be provided with a suitable cavity conceived to cooperate with an external pump for vacuum positioning the container in the space of the lower jaw.

In a still further embodiment of the container according to the invention, it further comprises a safety element for enabling an acute removal of the container from said space.

Preferably, the safety element is a cord which may be pulled for acutely removing the container from the oral cavity. Preferably, the cord comprises an indicator for indicating a position of the container and/or a condition of the electronic device. This has an advantage that a position and/or the condition of the monitor may be easily assessed from the outside without disrupting the monitoring sequence.

These and other aspects of the invention will be discussed in further detail with reference to drawings, wherein like reference numerals represent like elements. It will be appreciated that the drawings are presented for illustrative purposes and may not be used for limiting the scope of the appended claims. It will be further appreciated that while these exemplary embodiments are being discussed with reference to the U-shape they may be implemented on other shapes, such as O-shape and the arc-shape as well.

### BRIEF DESCRIPTION OF THE DRAWINGS

Figure 1 presents in a schematic way embodiments of the container according to the invention.
Figure 2 presents in a schematic way an embodiment of an outer slope of the container according to the invention.
Figure 3 presents in a schematic way a further embodiment of the container according to the invention.

### DETAILED DESCRIPTION OF THE DRAWINGS

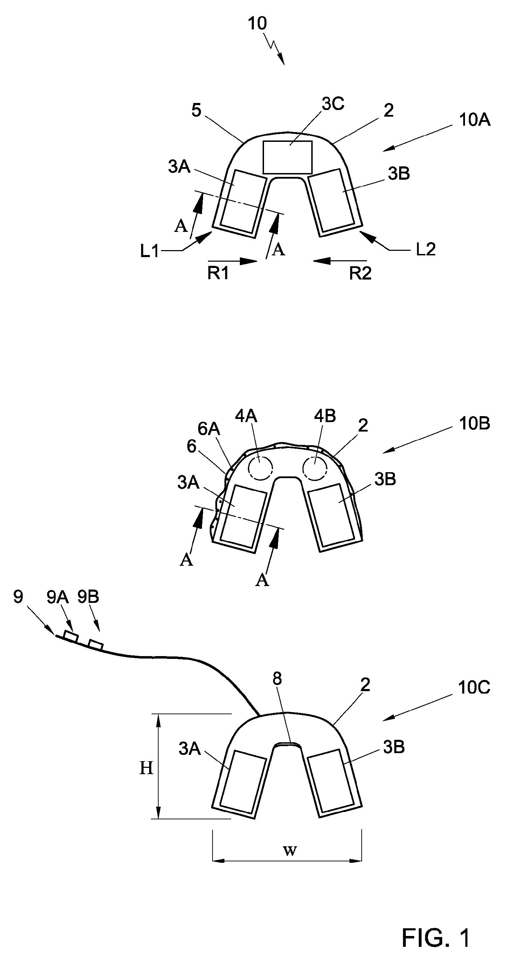

Figure 1 presents in a schematic way embodiments of the container 10 according to the invention. In view 10a a top view of the container according to the invention is shown. It will be appreciated that although the container 2 is depicted as a U-shape, other shapes are possible, like an O-shape an arc-shape or the like. It will be appreciated that in case the O-shape is used the container may be compressed by exercising a lateral force on the body of the O-shape at a desired position. The container 2 according to an aspect of the invention may comprise legs L1, L2 at least one which may be displaceable as is indicated by the arrows R1, R2. Accordingly, when the container is to be positioned inside the lower jaw of the recipient, it may be compressed by urging the one or two legs towards the symmetry axis of the U-shape (not shown). As a result handling of the container is improved. It will be appreciated that the container 10a may be handled by hand or using tweezers or any other suitable instrument. Preferably, for handling purposes the container is provided with a grip element, like a rim 8 shown in view 10c, a projection (not shown) or a cavity. For example, for this purpose it is also possible to use the pre-fabricated cavities 3a, 3b, 3c conceived to accommodate an electronic device for positioning purposes. Before or after positioning is completed, the cavities may be filled with desired electronics. Preferably, a temperature sensor, a photoplethysmograph or any other suitable device is provided in the cavities 3a, 3b or 3c. Those skilled in the art of intra-oral monitoring will readily appreciate what kind of electronic devices may be positioned inside the container according to the invention. It will be appreciated, however, that a suitable stimulating device, for example an electrode-based stimulator may also be positioned in the container according to the invention instead of, or next to a suitable monitor.

Alternatively or additionally, the electronic device may relate to a miniature pump, for example a squiggle pump, which may be used to collect a suitable volume of saliva for analysis. This technical measure is based on the insight that saliva comprises sufficient concentration of markers which may be a good indicator of physiologic condition of the patient. It will be further appreciated that the electronic pump may be provided in the intra-oral device of any shape and nature for enabling suitable physiologic monitoring. Preferably, the saliva pump is provided in the container according to the invention. It will be further appreciated that a suitable container may comprises one or more markers comprising suitable reagents conceived to undergo a chemical reaction with a suitable trace element in the saliva. Preferably, such marker is adapted to change color upon an event a target trace element is detected. This embodiment is particularly useful as it provides an immediate indication of the physiological condition of the patient and does not require substantive laboratory tests for analyzing collected saliva.

According to an aspect of the invention an outer surface 5 of the container 2 comprises a soft and, preferably, flexible material. This feature enables a better fixating of the container 2 in the space inside the lower jaw.

View 10b schematically depicts an embodiment of the container according to a further aspect of the invention wherein the soft material positioned at an outer surface of the container is shapeable on demand. For example, a suitable bag 6 may be arranged on an outer surface of the container. It will be appreciated that such bag may expand over the entire outer surface or over a portion of the outer surface of the container. The bag 6 may be filled with a suitable filling material, for example foam balls 6a, which may be brought into a desired shape under vacuum when the container is in place. This feature has an advantage that an individually shaped container may be provided on demand which may further improve accuracy of monitoring and user friendliness. For this purpose the vacuum ports 4a, 4b may be provided which are arranged in fluid communication with the bag 6. Alternatively or additionally the ports 4a, 4b may be arranged as cavities for enabling positioning of the container 2 in the lower jaw using vacuum suction.

View 10c schematically depicts a further embodiment of the container wherein only two cavities for accommodating electronic devices may be provided. It will be appreciated that one of the cavities may be used as a utility cavity, suitably accommodating a power supply and/or signal processing electronics, or wireless communication module. Advantageously, necessary wiring is embedded in the material of the container and runs from cavity 3a via the base portion to the cavity 3b. Other wiring arrangements are possible as well.

Optionally, the container according to the invention may comprise a safety element, like a cord 9, which may be used for emergency handling of the container, for example, when it is necessary to acutely remove the container from the intra-oral cavity. Advantageously, the safety element 9 is provided with suitable indicators 9a, 9b arranged to indicate that a position of the container is as desired and/or whether electronics functions properly. It will be appreciated that a former indicator may be a mark or the like, which the latter indicator may comprise an electronic means, like a LED lamp or the like.

According to a further aspect of the invention the container is dimensioned to a length W between 25 - 30 mm (i.e. the distance between the base of the U-shape and the distal portions of the legs). A width H of the U-shape may be set to a value in the range of 32 - 36 mm, when at rest. Preferably, a width of the compressed U-shape is 20 - 25 mm. It will be appreciated that for an O-shaped container the dimensions may be similar.

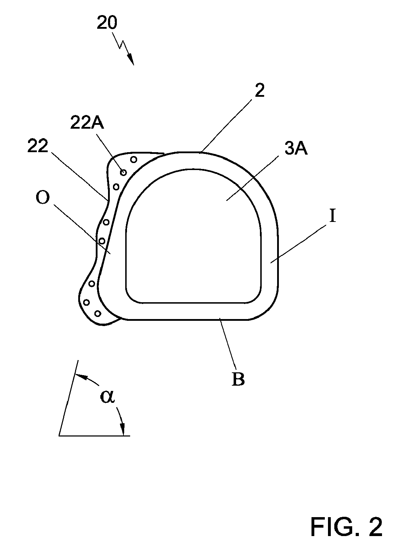

Figure 2 presents in a schematic way an embodiment of an outer slope of the container according to the invention. This view schematically indicates a cross-section of the container 2 taken along the line A-A as indicated in Figure 1. Accordingly, the outer surface O of the container 2 may be provided with a slope α for substantially matching an inner slope of the lower jaw cavity. It will be appreciated that an average slope may be selected based on clinical insights, or a set of containers may be provided each having a slightly different slope.

In addition, to improve fixation of the container in the lower jaw cavity, the outer surface of the container may be provided with a soft shapeable material. For example, a bag 22 may be provided, which is filled with suitable particles 22a. When the inner volume of the bag 22 is evacuated, the outer shape of the bag substantially conforms to the inner shape of the lower jaw enabling individually shaped container. This feature may still further improve functional features of the monitoring device associated with the container as well as user friendliness of the container. It will be further appreciated that in case when the shapeable material is provided the slope of the outer surface may be substantially right or even crossing the bottom portion B at an angle α > 90 degrees. This feature provides supplementary space for shaping the bag 22. The cavity 31 may be used for accommodating a suitable electronic meter or suitable supplementary equipment, like a power supply, a data processing unit, or a communication module.

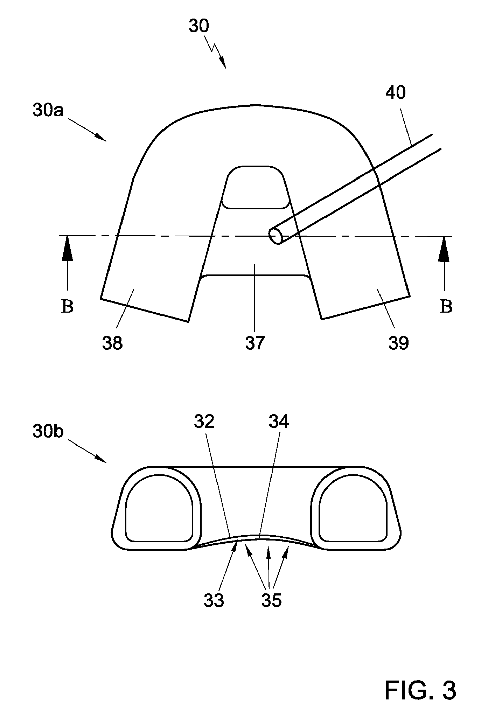

Figure 3 presents in a schematic way a further embodiment of the container 30 according to the invention. It will be appreciated that the container 30 may be implemented according to any of the aspect discussed with reference to Figure 1 or 2. In addition or alternatively, a bottom portion of the container 30, in the area between the legs 38 and 39, may be provided with a fixation member 37 (shown in view 30a), which may comprise two substantially thin layers of material (32, 34, shown in view 30b) provided with a chamber 33 therebetween wherein a lower layer of material is provided with perforations 35. Such fixation member may be used to fixate the container 30 against a bottom portion of the lower jaw of the patient under action of a vacuum pump 40 causing the chamber between these layers to be evacuated. It is appreciate that such construction causes generation of fixating force to the container 30 when the chamber 33 is at least partially evacuated. Preferably, for the bottom layer and/or for the upper layer a foil is used.

It will be appreciated that although specific embodiments of the structure according to the invention are discussed separately for clarity purposes, interchangeability of compatible features discussed with reference to isolated figures is envisaged. While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

## Claims

1. An intra-oral container comprising at least one region for accommodating an electronic device, wherein the container is curved and is shaped to be fitted in at least a portion of a space formed by a lower jaw of a recipient, wherein a portion of the container is displaceable for enabling compression of the container during insertion thereof in said space.

2. A container according to claim 1, wherein it is substantially U-shaped, O-shaped or arc-shaped.

3. A container according to claim 2, wherein the container is U-shaped having two legs and a base, wherein one or two legs are displaceable with respect to the base.

4. A container according to claim 2, wherein the container is U-shaped, the container further comprising a fixating member arranged between the legs and being formed of a top layer of material superposed on a bottom layer of material having chamber therebetween, wherein the bottom layer is perforated.

5. A container according to claim 3, wherein the at least one leg is resilient or wherein container is molded from a resilient material.

6. A container according to any one of the preceding claims, wherein the container is adapted to exhibit a lateral force on the lower jaw when positioned in said space.

7. A container according to any one of the preceding claims, wherein the container is substantially three-dimensional, a curvature of an outer surface of the container substantially matching a curvature of the space formed by the lower jaw.

8. A container according to any one of the preceding claims, wherein at least the outer surface of the container conceived to come into contact with the lower jaw comprises a soft material.

9. A container according to claim 8, wherein the soft material is thermoplastic.

10. A container according to claim 8, wherein the soft material comprises a bag provided with a filling, a shape of the bag being vacuum-shapeable.

11. A container according to any one of the preceding claim, wherein the container is provided with a projection, cavity or a rim for enabling handling of the container.

12. A container according to any one of the preceding claims, further comprising at least one cavity for enabling positioning of the container using vacuum suction.

13. A container according to any one of the preceding claims, further comprising a safety element for enabling an acute removal of the container from said space, or for indicating a position of the container and/or a condition of the electronic device.

14. A container according to any one of the preceding claim comprising an electronic device.

15. A container according to claim 14, wherein the electronic device is selectable from a group consisting of: a monitoring device, a stimulating device, a saliva pumping unit, or a camera.
